# EUROPÄISCHE PATENTSCHRIFT

(11) **EP 0 941 118 B1**
(45) Veröffentlichungstag und Bekanntmachung des Hinweises auf die Patenterteilung: **29.08.2001**
(21) Anmeldenummer: 97948874.9
(22) Anmeldetag: 04.11.1997
(51) Int. Cl.: A61K 39/12, C07K 16/08, A61K 39/42, A61K 39/395

(54) **PHARMAZEUTISCHE ANTIGEN-/ANTIKÖRPERPRÄPARATION**
PHARMACEUTICAL ANTIGEN-ANTIBODY PREPARATION
PREPARATION PHARMACEUTIQUE ANTIGENE-ANTICORPS

(30) Priorität: 05.11.1996 DE 19645559
(43) Veröffentlichungstag der Anmeldung: 15.09.1999
(73) Patentinhaber: Baxter Aktiengesellschaft, 1221 Wien (AT)
(72) Erfinder: EIBL, Martha, A-1180 Wien (AT); KREIL, Thomas, A-3400 Klosterneuburg (AT)
(74) Vertreter: HOFFMANN - EITLE
(86) Internationale Anmeldenummer: EP9706077
(87) Internationale Veröffentlichungsnummer: WO9819703

(56) Entgegenhaltungen:
- MURPHY ET AL.: "Effect of passive antibody on the immune response of cotton rats to purified F and G glycoproteins of respiratory syncytial virus (RSV)" VACCINE, Bd. 9, 1991, Seiten 185-189, XP002059577 in der Anmeldung erwähnt
- SCHUMACHER ET AL.: "Inhibition of immune responses against rabies virus by monoclonal antibodies directed against rabies virus antigens" VACCINE, Bd. 10, Nr. 11, 1992, Seiten 754-760, XP002059578 in der Anmeldung erwähnt
- GREEN ET AL.: "Depression of the immune response to an inactivated hepatitis a vaccine administered concomitantly with immune globulin" THE JOURNAL OF INFECTIOUS DISEASES, Bd. 168, 1993, Seite 740-743 XP002059579 in der Anmeldung erwähnt

## Beschreibung

Die vorliegende Erfindung betrifft Vakzine enthaltend als Komponenten: Flaviviridae-Antigene und gegen vom gleichen Virus kodierte Antigene gerichtete, protektive Antikörper, wobei zwischen den beiden Komponenten keine Immunkomplexe gebildet weden können.

Des weiteren wird deren Verwendung zur Herstellung eines Arzneimittels beansprucht.

Mikroben, wie Viren, Bakterien, Pilze oder Parasiten, können eine Vielzahl von Wirtsorganismen infizieren, pathologische Schädigungen hervorrufen und im Falle einer unkontrollierten Vermehrung ihren Wirt auch töten. Dem Immunsystem ist es zu verdanken, daß Erreger erkannt und effizient bekämpft werden.

In den letzten Jahren werden Prionen als neue infektiöse Pathogene der molekularen Art diskutiert. Prusiner S. et al., Advances in Virus Research, Vol. 29, pp 1-56, 1984, beschreiben die Krankheiten, die auf Prionen zurückgeführt werden, darunter Scrapie, Kuru, Creutzfeldt-Jakob Erkrankung, Gerstmann-Sträussler-Schenker Syndrom und familiäre Insomnia. Obwohl den Prionen eine molekulare Struktur zugeordnet wird, können entsprechende Nukleinsäuren, die für die Expression der Prionen verantwortlich sind, nicht ausgeschlossen werden. Die Kultivierung von Prionen *in vitro* bzw. die Infektion von Zell-Linien zur Gewinnung eines entsprechenden Titers wird von Race R. et al., Current Topics in Microbiology and Immunology, Vol. 172, pp 181-193, beschrieben.

Die Behandlung von Patienten, die dem Risiko einer Infektion aufgrund von Prionen bzw. "slow viruses" ausgesetzt sind, gewinnt zunehmend an Bedeutung. Die dafür zur Verfügung stehenden Tiermodelle und isolierten Moleküle können zur Testung von entsprechenden Behandlungsstrategien herangezogen werden.

Funktionell besteht das Immunsystem aus angeborenen, relativ unspezifischen Resistenzmechanismen einerseits, und erworbenen, sehr spezifischen Effektorfunktionen der Immunität. Bei den Effektormechanismen der Immunität ist weiterhin zwischen einer humoralen Immunantwort, also der Bildung von Antikörpern gegen Antigene eines Pathogens, und der zellulären Immunantwort zu unterscheiden.

Der Schutz gegen ein infektiöses Pathogen kann durch aktive oder passive Immunisierung erzeugt werden. Bei der passiven Immunisierung wird der humorale Schutz von einem Organismus, der diesen Schutz aufgebaut hat, auf einen anderen, naiven Organismus übertragen. Die Wirkung tritt sofort ein, die Dauer der Protektion ist allerdings beschränkt.

Aktive Immunität kann durch eine Infektion mit dem Pathogen induziert werden, andererseits aber auch durch Verabreichung eines Impfstoffes. Das Immunsystem wird bei diesen beiden Vorgängen aktiviert, was beispielsweise zur Proliferation von Antigen reaktiven T- oder B-Zellen führt, aus denen in weiterer Folge Gedächtniszellen entstehen. Die aktive Immunität bleibt daher über einen langen Zeitraum, auch jahrelang erhalten und kann, wenn nötig, durch eine Nachimpfung wieder aufgefrischt werden.

Für die aktive Immunisierung stehen zahlreiche Impfstoffe gegen eine Vielzahl infektiöser Pathogene zur Verfügung, und genauso vielfältig ist das Design solcher Vakzinen.

Folgende Arten von Impfstoffen sind derzeit vorwiegend in Verwendung: Ganzvirusvakzinen (als inaktivierte, abgetötete Vakzinen oder als attenuierte Lebendviren), gereinigte oder rekombinante Vakzinen, kodierend für Untereinheiten des Pathogens, sogenannte subunit Vakzinen, rekombinante Vektor-Vakzinen, synthetische Peptid-Vakzinen oder anti-idiotypische Vakzinen. Neuere Entwicklungen bei der Herstellung von Impfstoffen gehen z.B. in Richtung Verwendung "nackter" Nukleinsäuren (z.B. DNA Vakzinen).

Je nach Design der Vakzine und Form der Verabreichung ist eine unterschiedliche Art bzw. ein unterschiedliches Ausmaß der entsprechenden Immunreaktion zu erwarten. Ein häufig auftretendes Problem bei der Vakzinierung ist die geringe Immunogenität der verabreichten Vakzine bzw. das Problem, daß auch eine mehrfache Verabreichung zu keiner protektiven Immunität führt bzw. daß die induzierte Immunantwort keinen Schutz vor dem Pathogen bietet.

Aus diesen Gründen werden den Impfstoffen häufig sogenannte Immunstimulantien oder Adjuvantien zugesetzt. Solche Stoffe bewirken eine Verstärkung der Immunantwort (Immunpotentatoren), oder sie beeinflussen die Art der Immunantwort (Immunmodulatoren). Im Stand der Technik sind hierfür beispielsweise anorganische Stoffe, wie beispielsweise Aluminiumhydroxid, seit langem bekannt, ebenso wie die Verwendung von Wasser-in-Öl Emulsionen mit (komplettes Freund`sches Adjuvans) und ohne Mykobakterien (inkomplettes Freund`ches Adjuvans).

Auch verschiedene Zytokine, wie z.B. Interleukine oder Lymphokine, besitzen adjuvantierende Eigenschaften.

Von einigen Pathogenen, insbesondere von Viren, ist bekannt, daß nach Verabreichung einer entsprechenden Vakzine die Bildung neutralisierender Antikörper, welche normalerweise wünschenswert ist, zu einer erhöhten Infektivität des Pathogens führen kann. Dieses Phänomen ist als sogenanntes "antibody dependent enhancement of infectivity" bekannt (siehe z.B. Peiris J.S. and Porterfield J.S., 1979, Nature 282, 509-511).
Diesem Phänomen zufolge können bestimmte Antikörper gegen ein Virus auch zu einer Verschlechterung der klinischen Prognose beitragen, und ein Problem dieser Art sollte bei der Entwicklung eines Impfstoffes gerade vermieden werden.

Es ist somit notwendig, nach weiteren Strategien der Vakzinierung zu forschen, um auch in diesen Fällen einen wirksamen Schutz gegenüber dem Krankheitserreger, also gegenüber Infektion mit bzw. Erkrankung durch ein Pathogen, zu erreichen.

Solch eine neuere Strategie ist beispielsweise aus der WO 95 06 124 bekannt. In diesem Dokument wird beschrieben, daß nach Veränderung eines immundominanten Epitops eines Antigens ein hoher Antikörper Titer gegen nicht dominante Epitope des Antigens erzielt werden kann. Diese Veränderung (sogenannte "Immundämpfung") wurde durch eine gezielte Modifikation in den entsprechenden immundominanten Epitopen, beispielsweise durch Veränderungen in der Aminosäuresequenz, erreicht.

Galletti R. et al., 1995 (Vaccine, Vol.13, No.2, p.197-201) untersuchten die Immunantwort bei Verwendung von Vaccinia Rekombinanten, die Proteine des Masern Virus exprimieren, in Gegenwart passiv verabreichter Antikörper. Zu diesem Zwecke wurde einerseits polyklonales anti-Masern Serum und andererseits ein einzelner monoklonaler Antikörper gegen jeweils eines der rekombinanten Proteine gemeinsam mit den Vaccinia Rekombinanten verabreicht. In Abhängigkeit von der Antikörper Menge wurde eine Suppression der humoralen Immunantwort beobachtet.

Aus der WO 91 04 750 von Embrex Inc. ist eine Vakzine bestehend aus einem Lebendvirus und einem Virus neutralisierenden Faktor, der an das Lebendvirus gebunden vorliegt, bekannt. Der Virus-neutralisierende Faktor ist ein Antikörper oder ein Antikörper Fragment und muß in einer für die Neutralisierung des Virus ausreichenden Menge vorhanden sein. Der Virus-neutralisierende Faktor ist beispielsweise ein Antiserum, welches gemäß dem Schlüssel-Schloß-Prinzip genau gegen das Virus gerichtet ist.

Aus Pokric B. et al., 1993 (Vaccine, Vol.11, Nr.6,p.655-659)ist ein Immunkomplex bestehend aus "subunits" von NDV (Newcastle disease virus) und allogeneischen polyklonalen Virus neutralisierenden Antikörpern bekannt, der als antivirale Vakzine für Hühner verwendet werden kann. Die Autoren mutmaßen, daß sowohl die Inhibierung als auch die Stimulierung des Immunsystems durch Immunkomplexe in Zusammenhang mit dem Verhältnis von Antigen zu Antikörper steht, aber auch von der Art des Pathogens abhängig ist. Im Fall von HSV konnte beispielsweise nach Inokulierung von Kaninchen mit einem HSV - anti-HSV Immunkomplex, der in Gegenwart eines Überschusses Virus neutralisierender Antikörper gebildet worden ist, weder eine zelluläre noch eine humorale Immunantwort festgestellt werden.

Willett B.J. et al., 1994 (J.Immunol.Methods, 176, p.213-220) führten eine Immunisierung von Mäusen mit einem an eine feste Matrix gebundenen Komplex eines felinen CD4 Antigens mit einem monoklonalen anti-CD4 Antikörper (F117) und mit Formalin fixiertem Staphylococcus A durch. Die Autoren stellten fest, daß durch diesen Komplex in vivo verschiedene monoklonale Antikörper gegen feline Homologe von CD4 generiert werden können und schlußfolgern, daß felines CD4 somit wenigstens eine polymorphe Determinante enthalten muß.

Wenn bereits eine Infektion vorliegt oder der Zeitraum bis zu einer möglichen Exposition gegenüber einem Pathogen für die Ausbildung einer aktiven Immunität zu kurz ist, bietet eine passive Protektion die einzig denkbare Alternative.

Ideal wäre in solchen Fällen eine kombinierte aktive und passive Immunisierung, so daß einerseits ein sofortiger Schutz gegeben ist und andererseits die Möglichkeit besteht, eine langanhaltende Immunität zu induzieren.

Diese als Simultanimpfung bekannte Strategie wird auch heute noch für z.B. Hepatitis B, Tetanus oder Tollwut empfohlen (siehe Red Book, 1994, p.28). Bei Immunisierung mit attenuierten Viren wird dagegen von einer gleichzeitigen Gabe des entsprechenden Immunglobulins abgeraten (siehe hierzu Red Book 1994, p.28 bzw. p319).

In der neueren Literatur mehren sich jedoch die Hinweise darauf, daß auch bei Immunisierung mit einem Tot-Impfstoff die gleichzeitige Behandlung mit einem dafür spezifischen Immunglobulin, das sich zur Vakzine gemäß einem Schlüssel-Schloß Prinzip verhält, zu einer Reduktion sowohl des erzielten Titers an Impfantikörpern (siehe beispielsweise Schumacher C.L. et al., Vaccine, Vol.10 (11), 1992, p.754-760 oder Green M.S. et al., J.Infect.Dis., 1993, 168, p.740-3) als auch der erzielten Protektion (siehe beispielsweise Murphy B.R. et al., Vaccine, 1991, Vol.9, p.185-189 oder Murphy B.R. et al., J.Virol., 1988, Vol.62, No.10, p.3907-3910) führt.

Aufgabe der vorliegenden Erfindung ist es, eine Präparation zur Verfügung zu stellen, die die aus dem Stand der Technik bekannten Nachteile vermeidet und die auch einen verbesserten Impfstoff darstellt.

Es ist auch Aufgabe der vorliegenden Erfindung, die Immunantwort je nach Zusammensetzung der pharmazeutischen Präparation entsprechend modulieren zu können. Insbesondere soll die pharmazeutische Zusammensetzung eine verstärkte bzw. eine solche Immunität bei Mensch und Tier hervorrufen, daß beispielsweise einem "antibody dependent enhancement of infectivity" entgegengewirkt werden kann.

Weiters soll es mittels der erfindungsgemäßen Präparation möglich sein, herkömmliche Mechanismen, die beispielsweise bei der Simultanimpfung in einer reduzierten Immunogenität eines Impfstoffes resultieren, zu umgehen.

Eine weitere Aufgabe der vorliegenden Erfindung ist es, beispielsweise im Falle an sich pathologisch wirksamer Antigene eine Immuntoleranz gegenüber diesen Antigenen zu induzieren.

Die vorstehend genannte Aufgabe wird durch den Gegenstand der vorliegenden Patentansprüche gelöst.

Die erfindungsgemäßen Vakzine gemäß Patentanspruch 1 umfaßt ein Flaviviridae-Antigen, und mindestens einen Antikörper, der das Antigen bindet, wobei wenigstens ein Epitop des Antigens frei und immunogen ist.

Ausgewählte Viren sind aus der Familie der Flaviviridae vorzugsweise Enzephalitits Virus, wie FSME-Virus, oder Viren ausgewählt aus der Subgruppe der Dengue Viren oder Hepatitis C viren.

Unter einem Antigen in den erfindungsgemäßen Vakzine wird i.a. eine solche Substanz verstanden, die von dem lebenden Organismus als fremd erkannt wird und eine spezifische Immunantwort auslöst. Insbesondere ist unter Antigen auch eine Vielzahl von Antigenen zu verstehen.

Das Antigen kann ein Ganzpathogen sein, ein entsprechendes Fragment davon bzw. ein vom Pathogen kodiertes Antigen.

Das Antigen ist vorzugsweise ein Virus, es kann ein inaktives oder ein replikationsfähiges Virus sein.

Ein inaktives Virus ist beispielsweise ein inaktiviertes Virus oder ein Virusfragment. Die Inaktivierung erfolgt mittels hierfür gängiger Methoden, beispielsweise durch eine chemische Behandlung wie eine Behandlung mit einem Detergenz oder mit Formalin oder eine physikalische Behandlung, beispielsweise durch Hitze, Bestrahlung oder Ultraschall.

Ein attenuiertes Virus ist vorzugsweise derart attenuiert, daß es nicht mehr pathogen wirkt, jedoch den Wirt produktiv infizieren kann. Dieser Fall der produktiven Infektion ist so definiert, daß das Virus sich repliziert und zumindest teilweise wieder Viruspartikel bildet, ohne daß der Wirt erkrankt.

Das Antigen ist bevorzugterweise ausgewählt aus der Gruppe Peptid, Polypeptid, Protein, Nukleinsäure und zellulärer Bestandteil. Das Antigen kann auch ein entsprechendes Fragment oder Derivat sein. Auch Kombinationen davon sind möglich.

Ein Antigen in der erfindungsgemäßen pharmazeutischen Präparation ist beispielsweise ein strukturelles Protein, z.B. ein Kern- oder Hüllprotein eines Pathogens, es kann aber auch ein nicht strukturelles Protein sein, z.B. ein Stoff mit regulatorischen Eigenschaften, wie z.B. Reverse Transkriptase, RNA Polymerase, Integrase oder Protease.

Es werden auch entsprechende Vorläufermoleküle dieser Proteine sowie entsprechende Derivate mit einer vergleichbaren oder sogar verbesserten immunogenen Wirkung, wie z.B. (chemisch) modifizierte Proteine, Fragmente, Fusionsproteine, Mutanten, Analoge und dergleichen, erfindungsgemäß eingesetzt.

Die Derivate und insbesondere die Mutanten haben bevorzugterweise eine Aminosäuresequenz mit mindestens 80% Homologie zu dem entsprechenden natürlichen Protein.

Ein Antigen in den erfindungsgemäßen Vakzine kann auch ein solches sein, welches von dem Pathogen während des Reifungsprozesses, d.h. von verschiedenen Reifungsstadien, stammt. Das sind beispielsweise solche Antigene, deren Erscheinungsbild sich während der Replikation eines Pathogens verändert. Ein Beispiel für ein solches Antigen ist das bei Flaviviren auftretende preM Protein, welches dann zum M Protein prozessiert wird; Im reifen Virus kommt das preM Protein also nicht mehr vor.

In einer bevorzugten Ausführungsform ist das Antigen ein Gemisch mindestens zweier Antigene abgeleitet von einem Pathogen in verschiedenen Reifungsstadien.

Das Antigen in den erfindungegmäßen Vakzine enthält vorzugsweise ein protektives Epitop. Unter "protektivem Epitop" ist ein Epitop eines Antigens zu verstehen, das nach Verabreichung des Antigens an ein Lebewesen zu einer protektiven Immunantwort beiträgt. Eine protektive Immunantwort schließt beispielsweise einen ausreichenden Schutz vor einer Erkrankung nach einer Infektion des Lebewesen mit dem Pathogen diesem gegenüber ein. Dieser Schutz kann beispielsweise durch ein entsprechendes Tiermodell bzw. mit Hilfe eines eindeutig erwiesenen Surrogatmarkers bzw. mit einem entsprechenden Antikörpertiter nachgewiesen werden.

Auch ein kommerziell erhältlicher Impfstoff kann ein Antigen in der erfindungsgemäßen Vakzine darstellen.

Andererseits kann das Antigen in den erfindungsgemäßen Vakzine auch eine komplexe Mischung von Antigenen darstellen. Es handelt sich beispielsweise um eine Antigen-Zusammensetzung, welche wenigstens zwei voneinander verschiedene Antigene umfaßt. In den Vakziven sind somit vorzugsweise wenigstens zwei Antigene desselben Pathogens bzw. Antigene, die dieselben pathologischen Prozesse einleiten oder verursachen, enthalten.

Unter "Epitop des Antigens" ist jener Bereich eines Antigens zu verstehen, der präsentiert werden kann bzw. die Fähigkeit besitzt einen Immunreaktanten zu binden. Es handelt sich somit vorzugsweise um einen immunogen wirksamen Bereich des Antigens.

Das Epitop kann grundsätzlich ein immundominantes (vordergründiges) oder ein immunrezessives (hintergründiges, verdecktes) Epitop des Antigens sein. Das Epitop kann eine Sequenzdeterminante (=immunogener Abschnitt einer Peptidkette) oder eine Konformationsdeterminante (= immunogener Bereich, der sich aus der räumlichen Struktur ergibt) sein.

Das Antigen kann mittels hierfür gängiger Methoden hergestellt werden. Es kann beispielsweise aus einer Körperflüssigkeit oder einem Gewebeextrakt gewonnen werden, von einer rekombinanten Nukleinsäure kodiert werden oder von einer Zellkultur, insbesondere einer Säugerzellkultur, stammen.

Das Antigen oder ein Teil davon kann auch synthetisch hergestellt sein.

In den erfindungsgemäßen Vakzinen ist also mindestens ein Antikörper enthalten, der das Antigen bindet, wobei wenigstens ein Epitop des Antigens frei und imunogen ist.

In einer speziellen Ausführungsform sind die enthaltenen Antikörper solche mit verschiedenen Spezifitäten.

Insbesondere liegen die Antikörper in einer Zusammensetzung vor, in welcher die Antikörper gegen das freie immunogene Epitop des Antigens abgereichert bzw. nicht enthalten sind. Vorzugsweise enthält diese Zusammensetzung ein polyklonales Antiserum, in dem Antkörper gegen dieses Epitop des Antigens abgereichert bzw. nicht enthalten sind.

Die Abreicherung eines Antikörpers kann mittels hierfür gängiger Methoden erfolgen. Beispielsweise erfolgt die Abreicherung mittels Fällung, Dialyse, Zentrifugation, Adsorption, mittels eines chromatographischen Verfahrens, insbesondere mittels Immunaffinität, durch Verdünnen oder anderer hierfür gängiger Methoden. Die für die Chromatographie verwendete Matrix trägt beispielsweise eine für ein nicht exponiertes Epitop des Antigens analoge Struktur, und die im Eluat gewonnene Antikörper-Zusammensetzung ist dann an Antikörpern gegen dieses Epitop abgereichert. Auch der umgekehrte Fall, daß die Matrix eine für ein exponiertes Epitop eines Antigens analoge Struktur trägt, und die erhaltene Antikörper enthaltende Zusammensetzung dann an Antikörpern gegen exponierte Epitope abgereichert ist, ist möglich.

Ebenfalls möglich ist eine Anreicherung an einem bestimmten Antikörper durch beispielsweise entsprechende Zumischung.

Vorzugsweise ist in den erfindungsgemäßen Vakzinen der Antikörper spezifisch für ein exponiertes oder immundominantes Epitop des Antigens bzw. Pathogens. Somit ist ein verdecktes oder immunrezessives Epitop des Antigens frei und immunogen wirksam. Insbesondere ist der Antikörper ein bindender Antikörper und vorzugsweise ein neutralisierender Antikörper. Im allgemeinen ist es so, daß wenigstens ein Epitop des Antigens von dem Antikörper in der Zusammensetzung nicht erkannt wird bzw. nicht gebunden ist. In den erfindungsgemäßen Vakzinen liegt dann noch mindestens ein Epitop des Antigens mehr frei, als dies bei den aus dem Stand der Technik bekannten Komplexen der Fall ist.
Beispielsweise wird ein Virus oder ein Virusfragment zusammen mit einem Antiserum, das wenigstens einen Antikörper gegen ein Hüllprotein des Virus enthält, verabreicht. Eine protektive Immunantwort kann dann beispielsweise aufgrund der Bildung von Antikörpern, die gegen ein nicht strukturelles Protein des Virus gerichtet sind, erreicht werden. Im allgemeinen unterscheidet sich also das nach Verabreichung der Präparation gebildete Antiserum von der mit dem erfindungsgemäßen Vakzine verabreichten Antikörper-Zusammensetzung.

Antikörper können also als eine ein polyvalentes Antikörper-Gemisch enthaltende Zusammensetzung vorliegen, aber auch als ein Gemisch monoklonaler Antikörper oder aus polyklonalen Antikörpern.

Polyklonale Antikörper zeichnen sich insbesondere dadurch aus, daß sie bezüglich Spezifität, Affinität und Avidität unterschiedlich sind. Monoklonale Antikörper hingegen zeichnen sich durch gleiche Struktur, gleiche Spezifität, gleiche Affinität und gleiche Avidität aus.

Im Falle polyklonaler Immunglobuline werden diese von verschiedenen Zellen in vivo oder ex vivo gebildet, während monoklonale Antikörper in vitro von der klonalen Nachkommenschaft einer Zelle produziert werden.

Ein monoklonaler Antikörper kann auch gegen nur ein ganz spezifisches Epitop eines Antigens gerichtet sein.

Der Antikörper kann auch als ein Antikörper Fragment in der Zusammensetzung enthalten sein, wie z.B. als ein F(ab)₂ oder ein F(ab) Fragment. Vorzugsweise liegt der Fc-Teil des Antikörpers in freier Form vor.

Der Antikörper ist beispielsweise ein natürliches oder synthetisches Polypeptid oder Protein, mit der Fähigkeit ein entsprechendes Epitop eines Antigens zu erkennen und ggfs. zu binden.

Der Antikörper kann auch ein chimärer, insbesondere humanisierter Antikörper sein, welcher beispielsweise durch rekombinante DNA-Klonierung erhältlich ist.

Der Antikörper kann ein Immunglobulin vom Typ IgM, IgG, IgA, IgD oder IgE, vorzugsweise in nativer Form, sein. Bevorzugterweíse sind die Immunglobuline vom Typ IgG und IgA, am meisten bevorzugt vom Typ IgG.

Ist in den erfindungsgemäßen Vakzinen das Antigen in Form von mehreren Antigenen enthalten, so sind vorzugsweise alle Epitope eines Antigens durch die Antikörper abgedeckt, und mindestens ein Epitop eines zweiten Antigens ist immunogen.

Es ist also beispielsweise möglich, eine Immunantwort gegen ein solches Epitop des Antigens zu induzieren, gegen welches ohne Vorliegen der entsprechenden Antikörper keine bzw. eine nur geringe Immunantwort induziert werden könnte. Mittels der erfindungsgemäßen pharmazeutischen Präparation kann somit die Immunogenität eines bestimmten Epitops bzw. eines bestimmten Antigens gesteuert bzw. verstärkt werden.

Im allgemeinen wird durch die Vakzine wenigstens ein Epitop des Antigens durch die Antikörper abgedeckt und die Immunogenität eines anderen, nicht abgedeckten Epitops dadurch erhöht. Beispielsweise kann eine unerwünschte Immunantwort gegen ein immundominantes Epitop des Antigens vermindert werden, da ein immunrezessives Epitop des Antigens dominierend werden kann und die gewünschte Immantwort verstärkt werden.

Der Antikörper kann in einer Zusammensetzung vorliegen, in der weiters Liganden enthalten sind (Liganden-Zusammensetzung).
Die Liganden-Zusammensetzung kann verschiedene Liganden für das Pathogen bzw. Antigen enthalten; in einer bevorzugten Ausführungsform enthält sie mehr als zwei verschiedene Liganden.

Das Vorliegen weiterer bzw. verschiedener Liganden hat den Vorteil, daß unterschiedliche Bindungen mit dem entsprechenden Antigen bzw. Pathogen ermöglicht werden. Dies kann gerade im Falle des Vorliegens replizierender Pathogene oder im Falle von Pathogenen, die zur Bildung sogenannter Escape-Mutanten neigen, mit größerer Sicherheit die Infektiösität des Pathogens bzw. die Pathogenese verhindern und insgesamt die Sicherheit der jeweiligen Vakzine erhöhen.

Aufgrund der Verschiedenheit der Liganden wird im Falle der Verwendung der erfindungsgemäßen Vakzine für die Simultan-impfung auch eine erhöhte Sicherheit in der Protektivität erreicht. Aufgrund der Unterschiedlichkeit betreffend beispielsweise Halbwertszeit des jeweiligen Liganden oder Unterschiede in der Avidität ist eine sofortige und persistente Bindung des Pathogens bzw. Antigens mit den Liganden möglich.

Der Ligand ist vorzugsweise ausgewählt aus der Gruppe Rezeptor, Rezeptor Fragment und Chemikalie. Auch Kombinationen davon sind möglich.

Die Verschiedenheit kann beispielsweise in der Art, Klonalität und/oder Spezifität des Liganden begründet sein.

Unter Spezifität wird die Selektivität eines Liganden für das entsprechende Antigen verstanden.

Vorzugsweise handelt es sich bei dem Liganden um einen solchen, der natürlicherweise vorkommt bzw. handelt es sich um einen nativen Liganden. Der Ligand kann aber auch ein entsprechendes Analogon sein, oder er ist synthetisch hergestellt. Jedenfalls weist er eine starke Bindung zu dem Pathogen bzw. Antigen auf.

Die Zusammensetzung enthält vorzugsweise Antikörper, Antikörperfragmente bzw. Liganden humanen Ursprungs oder rekombinante bzw. humanisierte.

Die Herstellung der Antikörper, Antikörperfragmente bzw. Liganden erfolgt mittels aus dem Stand der Technik bekannter Methoden (siehe beispielsweise Maniatis T. et al., Molecular Cloning, A Laboratory Manual, 1983).

In den erfindungsgemäßen Vakzinen ist kein Immunkomplex enthalten.

In einer bevorzugten Ausführungsform ist das Antigen ein nicht strukturelles Protein eines Pathogens und der Antikörper gerichtet gegen ein strukturelles Protein des Pathogens.
In einer weiteren bevorzugten Ausführungsform ist umgekehrt das Antigen ein strukturelles Protein des Pathogens und der Antikörper gerichtet gegen ein nicht strukturelles Protein des Pathogens.

Der Antikörper ist in einer weiteren bevorzugten Ausführungsform gegen ein Oberflächen-Protein des Pathogens gerichtet.

Der Antikörper ist in einer bevorzugten Ausführungsform in einer Zusammensetzung enthalten, die protektiv gegen eine Infektion mit und/oder eine Erkrankung durch das Pathogen ist.
Eine protektive Zusammensetzung ist eine solche, die auch bei alleiniger Verabreichung einen ausreichenden Schutz vor einer Infektion mit dem Pathogen bzw. vor einer entsprechenden Infektionskrankheit bewirkt. Vorzugsweise liegen damit in der Zusammensetzung protektive Antikörper vor.

Sowohl Antigen als auch Antikörper sowie ggfs. vorhandene Liganden liegen bevorzugterweise nicht nur in isolierter, sondern auch in gereinigter Form vor. Insbesondere liegen das Antigen bzw. der Antikörper und ggfs. vorhandene Liganden in reiner bzw. angereicherter Form vor.

Für die Reinigung werden hierfür gängige Methoden angewandt.
Eine solche Reinigung kann beispielsweise nach Größe, nach Ladung oder nach Löslichkeit erfolgen.

Beispielsweise erfolgt die Reinigung von Antigen bzw. Antikörper bzw. Ligand gemeinsam oder separat durch Filtration, Zentrifugation (z.B. Dichtegradientenzentrifugation), Dialyse/Diafiltration, chemische Fällung oder mittels chromatographischer Verfahren insbesondere durch Gelchromatographie, Affinitätschromatographie, hydrophobe Chromatographie oder "reversed phase" Chromatographie. Damit werden v.a. kontaminierende Materialien des Ausgangsmaterials, wie Proteine eines Zellkulturüberstandes oder Immunglobulinaggregate, auf ein pharmazeutisch akzeptables Maß abgereichert.

Des weiteren können auch Schritte zur Inaktivierung von gegebenenfalls vorhandenen infektiösen Partikel, beispielsweise von Viren, durchgeführt werden.

Solche Verfahren zur Inaktivierung können eine Beschallung, Betrahlung, wie z.B. radioaktive Bestrahlung oder eine Bestrahlung mit sichtbarem oder ultraviolettem Licht, eine chemische Behandlung, Hitzebehandlung, Enzymbehandlung oder eine Behandlung mit einem Detergenz umfassen. Die einzelnen Verfahren können auch kombiniert werden. Ebenso können physikalische Verfahren zur Abreicherung von viralen Partikeln eingesetzt werden, insbesondere die Nanofiltration.

In einer bevorzugten Ausführungsform die Vakzine enthälten gegebenenfalls weiters einen als Adjuvans wirkenden Stoff.

Das erfindungsgemäße Vakzine ist beispielsweise zur Behandlung von Infektionen, z.B. Enzephalitis.

Sowohl die pharmazeutische Präparation, die Antikörper als auch die Vakzine können in lyophilisierter Form vorliegen und gelagert werden.

Das erfindungsgemäße Vakzine wird auch als ein Set zur Verfügung gestellt, wobei das Set das Antigen und den Antikörper bzw. die Zusammensetzung in separaten Behältern umfaßt.

Antigen und Antikörper bzw. Zusammensetzung liegen dabei in einer für die simultane, sequentielle oder parallele Verabreichung geeigneten Form vor.

Bevorzugt liegt dieses Set entweder als Kombination oder als Einzelkomponenten in einer für die parenterale Verabreichung geeigneten Form vor, beispielsweise das Antigen i.m. und der Antikörper i.v. Die i.v. verträglichen Antikörper sind von besonderer Qualität.

Darüber hinaus besteht auch die Möglichkeit, das Set oder lediglich ein Teil davon, also das Antigen oder der Antikörper bzw. die Zusammensetzung, in einer für die mukosale, beispielsweise orale, Verabreichung geeigneten Form vorliegt.
Die Präparate können also in Lösung, Suspension oder als feste Präparation, z.B. als Tablette oder als Suppositorium, in einer Fertigspritze oder als Spray zur Verfügung gestellt werden.

Die Stabilität der Präparate macht es auch möglich, diese über einen Zeitraum von mindestens 2 Jahren zu lagern.

Die für die Verabreichung an ein Tier oder an einen Menschen geeignete Menge bzw. Dosis muß je nach gewünschter Wirkung in präklinischen oder klinischen Versuchen ermittelt werden. Für die Präklinik können entsprechende Tiermodelle herangezogen werden.

Beispielsweise wird je nach Affinität oder Avidität der vorliegenden Antikörper, eine Dosis in einem Bereich von 1 µg bis 1000 mg pro kg Körpergewicht verabreicht. Bei Vorliegen hochaffiner Antikörper werden diese vorzugsweise in einer Dosis zwischen 0,1 bis 10 mg pro kg Körpergewicht verabreicht. Liegen Antikörper mit geringerer Affinität vor, so liegt die zu verabreichende Dosis vorzugsweise zwischen 10 bis 1000 mg pro kg Körpergewicht.

Bei Verwendung eines Ganzvirus als Antigen wird dieses beispielsweise in einer Dosis zwischen 1 µg bis 1 mg pro kg Körpergewicht verabreicht.

Das erfindungsgemäße Vakzine ist insbesondere zur Modulation einer Immunantwort, v.a. durch Beeinflussung des humoralen Immunsystems, geeignet. Insbesondere kann bei entsprechender Wahl der vorliegenden Antikörper bzw. Liganden eine Immunantwort gegen ein solches Epitop des Antigens induziert werden, wogegen mittels einer entsprechenden Antikörper-Präparation ohne diese Antikörper bzw. Liganden keine oder keine ausreichende Immunantwort erzielt werden kann. Mittels der erfindungsgemäßen Vakzine kann also insbesondere eine protektive Immunantwort gefördert werden.

Eine Modulation der Immunantwort kann auch dahingehend erfolgen, daß die Immunantwort gegen ein an einen Antikörper gebundenes Epitop des Antigens bzw. Pathogens.

Durch die Bindung an einen Antikörper kann also beispielsweise gegen ein Epitop des Antigens, welches eine pathogenetische Reaktion hervorruft, eine reduzierte Reaktionsbereitschaft induziert werden. Diese Immuntoleranz hilft einem Organismus, im speziellen einem Patienten, eine Überreaktion gegen bestimmte Antigene wie Allergene zu behandeln.

Im speziellen Falle, daß Antikörper und gegebenenfalls Liganden vorliegen protektiv wirksam sind, ist die erfindungsgemäße Vakzine überraschenderweise zur Vermittlung einer sofortigen, kontinuierlichen und persistenten Protektion gegen eine durch das Pathogen hervorgerufene Erkrankung und/oder eine Infektion mit dem Pathogen mittels Simultanimpfung geeignet. Insbesondere hat sich gezeigt, daß die aus dem Stand der Technik bekannten Probleme dadurch vermieden werden können.

Die folgenden Beispiele sollen die Erfindung noch näher erläutern, ohne diese jedoch darauf zu beschränken.

### Beispiel 1:

### a) Passive Immunisierung

BALB/c Mäuse wurden intravenös mit Maus-Seren behandelt. Eines dieser Seren enthielt polyklonale, monospezifische Antikörper gegen Glykoprotein E (gE), das Oberflächenprotein von FSME-Virus, ein anderes Serum polyklonale, monospezifische Antikörper gegen NS1, ein Nichtstrukturprotein das vom FSME-Genom kodiert wird und im Viruspartikel nicht vorhanden ist, also auch nicht in der für die aktive Immunisierung verwendeten FSME-Ganzvirus-Vakzine.

### b) Aktive Immunisierung

Unbehandelte und mit anti-gE oder anti-NS1 Serum behandelte Mäuse wurden in einem Intervall von 4 Wochen zweimal mit einer inaktivierten FSME Ganzvirus Vakzine (FSME-IMMUN® Inject, Immuno AG) immunisiert, wobei die erste Dosis im Falle vorangegangener Verabreichung von Immunglobulin 2 Stunden nach dieser gegeben wurde.

### c) Ermittlung der Protektion gegen FSME

Die Effizienz der passiven Immunisierung wurde durch "challenge" der Mäuse mit 100 LD₅₀ FSME-Virus (Stamm Neudörfl, propagiert in Vero Zellen, Immuno AG), also einer hoch-letalen Dosis an Virus, getestet. Der Tod nach einer solchen FSME-Virus Infektion tritt im Mittel nach etwa 10 Tagen ein, daher wurden Tiere die 4 Wochen nach dem "challenge" gesund waren als Überlebende gewertet.

Die Effizienz der aktiven Immunisierung mit Vakzine allein bzw. in Kombination mit dem jeweiligen Immunglobulin wurde folgendermaßen bestimmt:
10 Wochen nach der ersten Vakzinierung wurde ein "challenge" der Mäuse mit 100 LD₅₀ FSME-Virus durchgeführt und die Anzahl überlebender Mäuse wie beschrieben festgestellt. Außerdem wurden Titer von FSME-Virus spezifischen IgG Antikörpern mittels ELISA bestimmt.

Zum Zeitpunkt des "challenge", 10 Wochen nach der ersten Immunisierung, war die passive, durch Antikörper vermittelte Protektion bereits auf ein nicht nachweisbares Niveau abgefallen, d.h. Tiere, die nur mit einem der beiden Seren behandelt worden sind, waren zum Zeitpunkt des "challenge" nicht mehr gegen FSME geschützt.

### d) Ergebnisse

Injektion von Antiseren gegen gE oder NS1 schützte die verwendeten Versuchstiere verläßlich gegen FSME. Der erzielte Schutz setzte sofort ein, da keines der Tiere, die zwei Stunden nach der Behandlung einer massiven FSME-Virus Exposition unterzogen wurden, an FSME erkrankte, während alle unbehandelte Kontrolltiere an FSME verstarben. Allerdings war dieser passive Schutz nur von relativ kurzer Dauer: Zehn Wochen nach der Behandlung mit gE oder NS1 Antiseren waren die Versuchstiere nicht mehr gegen eine FSME-Virus Exposition geschützt.

Zweimalige Immunisierung von Versuchstieren mit einer FSME-Ganzvirus Vakzine führte zu aktiver Immunität, und diese aktive Immunität schützt alle Versuchstiere gegen die vorher beschriebene FSME-Virus Exposition. Im Gegensatz zu einer passiven Immunisierung durch Transfer von Antikörpern ist die durch aktive Immunsierung erzielte Protektion lang anhaltend.

Bei simultaner Verabreichung der Vakzine und eines anti-gE Serums waren die Tiere zwar gegen eine sofortige FSME-Virus Belastung passiv geschützt, der Erfolg der aktiven Immunisierung nach zweimaliger Impfung war jedoch deutlich vermindert: Zehn Wochen nach der ersten Immunisierung überlebten nur etwa 50% der Tier eine FSME-Virus Exposition, während alle Tiere die nur mit der Vakzine behandelt worden waren das "challenge" überlebten. Bei gleichzeitig mit gE Antikörpern und Vakzine behandelten Tieren wurden im ELISA auch deutlich niedrigere Antikörper Titer gegen FSME-Virus als bei nur immunisierten Tieren festgestellt.

Im Gegensatz zu dieser reduzierten Immunogenität im Fall der Kombination von Vakzine und anti-gE Antikörpern waren die Mäuse, die Vakzine und anti-NS1 Serum erhielten, sofort und lang anhaltend gegen eine FSME-Virus Exposition geschützt. Die gemessenen ELISA Titer an FSME-Virus Antikörper von aktiv immunisierten oder von aktiv und passiv immunisierten Tieren waren vergleichbar.

Diese Ergebnisse zeigen eindrucksvoll, daß die Verabreichung der erfindungsgemäßen Präparation umfassend als eine Komponente eine protektive Zusammensetzung einerseits zu einer unmittelbaren Protektion gegen eine Virus Infektion führt, und andererseits auch eine effiziente aktive Immunität induziert wird, welche lang anhaltend ist. Es kann dadurch ein sofortiger und persistierender Schutz erreicht werden.

### Beispiel 2:

Als Antigen wurde BALB/c Mäusen intraperitoneal replikationsfähiges FSME-Virus (gereinigt aus dem Kulturüberstand von infizierten VERO-Zellen) injiziert, und zwar zwei Stunden nach subkutaner Applikation von FSME-Virus Antikörpern. Die verwendeten FSME-Virus Antikörper waren dabei entweder nur spezifisch für Glykoprotein E (gE), das Oberflächenprotein von FSME-Virus, (FSME-BULIN® , Immun AG), oder für NS1, ein nicht-strukturelles Protein das vom FSME-Virus Genom kodiert wird (Mausserum). Antikörper beider Spezifitäten schützen die so behandelten Versuchstiere gegen FSME. Nach zehn Wochen, zu einem Zeitpunkt an dem die passiv administrierten Antikörper in den Versuchstieren nicht mehr nachweisbar sind, wurden die Antikörper in den Seren der Versuchstiere auf FSME-Virus spezifische Reaktivitäten im Western Blot untersucht. Als Antigen für den Western Blot wurden dabei Lysate von FSME-Virus infizierten Zellen verwendet, also eine Präparation die alle von FSME-Virus kodierten Proteine enthält.

Dabei wurde gefunden, daß die Antikörper von Seren jener Tiere, die ursprünglich mit anti-gE Antikörpern behandelt worden waren, prädominant mit dem immunologisch wichtigsten nicht-strukturellen Protein von FSME-Virus, NS1, reagierten, während bei anti-NS1 behandelten Tieren die Reaktion mit dem Oberflächenprotein gE von FSME-Virus am deutlichsten war.

Dies läßt den Schluß zu, daß die Immunogenität von FSME-Virus Proteinen bei gleichzeitiger Anwesenheit von Antikörpern gegen diese Proteine deutlich reduziert ist, während die Immunogenität anderer Virus-kodierter Proteine vergleichsweise verbessert wird.

Die beschriebene Methode erlaubt daher die Modulation der Immunantwort gegen verschiedene Proteine - wie im Beispiel beschrieben - oder aber auch gegen verschiedene Epitope auf einem Protein. Bei Infektion mit Erregern, deren potentiell protektive Antigene nicht immundominant sind, kann im Sinne der Erfindung die Immunogenität eines dominanten Antigens mittels Gabe von dafür spezifischem Immunglobulin reduziert werden, und damit gleichzeitig erreicht werden, daß die Immunantwort gegen ein zuvor verhältnismäßig weniger immunogenes Epitop des Erregers verbessert wird.

Erfindungsgemäß ist es außerdem möglich, die Immunantwort gegen zum Beispiel das Antikörper-abhängige Verstärken einer Infektion, sog. "enhancement", begünstigende Determinanten eines Pathogens durch Gabe von dafür spezifischen Antikörpern zu reduzieren.

## Patentansprüche

1. Vakzine enthaltend als Komponente zur aktiven Immunisierung
Flaviviridae-Antigene und als Komponente zur passiven Immunisierung mindestens einen protektiven Antikörper gegen vom gleichen Virus kodierte Antigene die jedoch nicht in der Komponente zur aktiven Immunisierung enthalten sind, wobei der Antikörper mit den Flaviviridae-Antigenen keine Immunkomplexe bilden kann.

2. Vakzine nach Anspruch 1, **dadurch gekennzeichnet**, daß die Flaviviridae-Antigene nicht-strukturelle Proteim des Virus sind und Antikörper gegen strukturelle Proteine des Virus gerichtet sind.

3. Vakzine nach Anspruch 1, **dadurch gekennzeichnet**, daß die Flaviviridae-Antigene strukturelle Antigene des Virus sind und Antikörper gegen nicht-strukturelle Proteine des Virus gerichtet sind.

4. Vakzine nach einem der Ansprüche 1 bis 3, **dadurch gekennzeichnet**, daß die Flaviviridae-Antigene ausgewählt sind. aus der Gruppe Ganzvirus oder Virus Fragment.

5. Vakzine nach einem der Ansprüche 1 bis 4, **dadurch gekennzeichnet**, daß die Flaviviridae-Antigeneein attenuiertes, ein inaktives oder ein inaktiviertes Virus ist.

6. Vakzine nach einem der Ansprüche 1 bis 5, **dadurch gekennzeichnet**, daß Antigere und Antikörper in gereinigter Form enthalten sind.

7. Vakzine nach einem der Ansprüche 1 bis 6, **dadurch gekennzeichnet**, daß sie als ein Set zur Verfügung gestellt wird, welches Antigen und Antikörper in separaten Behältern umfaßt.

8. Vakzine nach Anspruch 7, **dadurch gekennzeichnet**, daß Antigen und Antikörper in einer für die simultane, sequentielle oder parallele Verabreichung geeigneten Form vorliegen.

9. Verwendung einer Vakzine nach einem der Ansprüche 1 bis 8, zur Herstellung eines Arzneimittels zur Vermittlung einer sofortigen kontinuierlichen und persistenten Protektion gegen eine Flaviviridae-Infektion.

10. Verwendung einer Vakzine nach einem der Ansprüche 1 bis 8, zur Herstellung eines Arzneimittels für die Simultanimpfung gegen eine Flaviviridae-Infektion.

## Claims

1. Vaccine containing as the component for active immunisation against flaviviridae antigens and as the component for passive immunisation, at least one protective antibody against antigens coded by the same virus, but which are not present in the component for active immunisation, wherein the antibody cannot form immunocomplexes with the flaviviridae antigens.

2. Vaccine according to claim 1, **characterised in that** the flaviviridae antigens are non-structural proteins of the virus and antibodies are directed to structural proteins of the virus.

3. Vaccine according to claim 1, **characterised in that** the flaviviridae antigens are structural antigens of the virus and antibodies are directed to non-structural proteins of the virus.

4. Vaccine according to one of claims 1 to 3, **characterised in that** the flaviviridae antigens are selected from the group comprising whole virus or virus fragment.

5. Vaccine according to one of claims 1 to 4, **characterised in that** the flaviviridae antigen is an attenuated, an inactive or an inactivated virus.

6. Vaccine according to one of claims I to 5, **characterised in that** antigens and antibodies are present in purified form.

7. Vaccine according to one of claims 1 to 6, **characterised in that** it is provided as a set which comprises antigen and antibody in separate containers.

8. Vaccine according to claim 7, **characterised in that** antigen and antibody are present in a form suitable for simultaneous, sequential or parallel administration.

9. Use of a vaccine according to one of claims 1 to 8, for producing a medicament for imparting immediate continuous and persistent protection against a flaviviridae infection.

10. Use of a vaccine according to one of claims 1 to 8, for producing a medicament for simultaneous inoculation against a flaviviridae infection.

## Revendications

1. Vaccin contenant, en tant que composant d'immunisation active, des antigènes flaviviridae et, en tant que composant d'immunisation passive, au moins un anticorps protecteur contre des antigènes codés par le même virus, qui cependant ne sont pas contenus dans le composant d'immunisation actif, l'anticorps ne pouvant former aucun complexe immun avec les antigènes flaviviridae.

2. Vaccin selon la revendication 1, **caractérisé en ce que** les antigènes flaviviridae sont des protéïnes non structurelles du virus et des anticorps sont dirigés contre les protéïnes structurelles du virus.

3. Vaccin selon la revendication 1, **caractérisé en ce que** les antigènes flaviviridae sont des antigènes structurels du virus et des anticorps sont dirigés contre les protéïnes non structurelles du virus.

4. Vaccin selon l'une des revendications 1 à 3, **caractérisé en ce que** les antigènes flaviviridae sont choisis dans le groupe d'un virus complet ou d'un fragment de virus.

5. Vaccin selon l'une des revendications 1 à 4, **caractérisé en ce que** les antigènes flaviviridae est un virus atténué, inactif ou inactivé.

6. Vaccin selon l'une des revendications 1 à 5, **caractérisé en ce que** les antigènes et les anticorps sont contenus sous forme épurée.

7. Vaccin selon l'une des revendications 1 à 6, **caractérisé en ce qu**'il est fourni sous la forme d'un kit ou set, englobant l'antigène et l'anticorps dans des récipients séparés.

8. Vaccin selon la revendication 7, **caractérisé en ce que** l'antigène et l'anticorps se présentent sous une forme convenant pour l'administration simultanée, séquentielle ou parallèle.

9. Utilisation d'un vaccin selon l'une des revendications 1 à 8, pour la préparation d'un médicament pour conférer une protection immédiate, continue et persistante contre une infecion par flaviviridae.

10. Utilisation d'un vaccin selon l'une des revendications 1 à 8, pour la préparation d'un médicament pour la vaccination simultanée contre une infection flaviviridae.
